# EUROPÄISCHE PATENTSCHRIFT

(11) **EP 1 313 405 B1**
(45) Veröffentlichungstag und Bekanntmachung des Hinweises auf die Patenterteilung: **31.03.2004**
(21) Anmeldenummer: 01962600.1
(22) Anmeldetag: 10.08.2001
(51) Int. Cl.: A61B 18/20

(54) **MEDIZINISCHES LASERBEHANDLUNGSMODUL**
MEDICAL LASER TREATMENT MODULE
MODULE DE TRAITEMENT LASER MEDICAL

(30) Priorität: 23.08.2000 DE 10041421
(43) Veröffentlichungstag der Anmeldung: 28.05.2003
(73) Patentinhaber: Meister, Jörg, 41238 Mönchengladbach (DE); Gutknecht, Norbert, 52159 Roetgen (DE); Apel, Christian, 52076 Aachen (DE)
(72) Erfinder: Meister, Jörg, 41238 Mönchengladbach (DE); Gutknecht, Norbert, 52159 Roetgen (DE); Apel, Christian, 52076 Aachen (DE)
(74) Vertreter: Jostarndt, Hans-Dieter, Dr. Dipl.-Phys.
(86) Internationale Anmeldenummer: PCT/DE2001/002958
(87) Internationale Veröffentlichungsnummer: WO 2002/015808

(56) Entgegenhaltungen:
- EP-A- 0 867 151
- DE-A- 2 352 670
- DE-A- 4 029 530
- DE-A- 4 030 734
- US-A- 4 808 789
- US-A- 4 862 888
- US-A- 5 196 004
- US-A- 5 249 192
- US-A- 5 295 143

## Beschreibung

Die Erfindung betrifft ein medizinisches Laserbehandlungsmodul nach dem Oberbegriff des Anspruchs 1.

Lasersysteme stellen sowohl in der Technik, in der Materialbearbeitung als auch in der Medizin unverzichtbare Werkzeuge dar. Sie ermöglichen präzises, punktgenaues und berührungsloses Arbeiten ohne mechanische Verschleißteile wie z.B. Sägeblätter oder Bohrer.

Für medizinische Anwendungen gibt es eine Vielzahl von Lasersystemen. Von zentraler Bedeutung ist hierbei für jeden Laser sein aktives Medium, welches die Emissionswellenlängen vorgibt und somit das Einsatzgebiet des Lasers in der Medizin festlegt. Diese Auswahl wird im Wesentlichen durch die wellenlängenabhängige Absorption der Laserstrahlung im Gewebe getroffen.

Verschiedene Lasersysteme werden sowohl in der Humanmedizin wie zum Beispiel der Augenheilkunde, Dermatologie, plastischen Chirurgie, Gynäkologie, Neurochirurgie, Urologie und Zahnmedizin als auch in der Tiermedizin eingesetzt. Ein Beispiel ist die Behandlung von Fehlsichtigkeit mittels Eximerlaser, deren Emissionsspektrum im Ultravioletten liegt, zur Korrektur der Hornhautform durch Abtragen kleinster Gewebeteile. Auch bei der Behandlung des grauen Stars oder des grünen Stars (Glaukom) werden Laser eingesetzt. Bei der Behandlung des grünen Stars wird die Regulierung des Augeninnendruckes wiederhergestellt. In der Zahnmedizin wird der Laser beispielsweise zur Behandlung von Parodontose und Zahnfleischerkrankungen sowie als Bohrer-Ersatz eingesetzt.

Grundlage der Erzeugung von Laserstrahlung ist immer die stimulierte Emission, deren Prozess erstmals von Albert Einstein beschrieben wurde. Durch eine Anregung der Atome, beziehungsweise der Moleküle im laseraktiven Medium erfolgt eine Besetzung höher gelegener Energieniveaus, welche für den Laserübergang verantwortlich sind. Ist die Anregung stark genug, um eine Übervölkerung des oberen Laserniveaus zu erzeugen (pumpen), spricht man von einer Besetzungsinversion. Letztendlich kommt es, als Folge eines spontanen Emissionsüberganges zur stimulierten Emission, das heißt zu einer künstlich generierten Entvölkerung des oberen Laserniveaus zur Abstrahlung von Laserstrahlen.

Das Verfahren, mit dem das Lasermedium angeregt wird, hängt vom verwendeten Lasermedium ab. Die drei wesentlichen Anregungsarten sind:
i) Gasentladung, das heißt Plasmabildung bei Gaslasern.
ii) Optisches Pumpen bei Festkörperlasersystemen.
iii) Elektrisches Pumpen bei Diodenlasern.

Von zentraler Bedeutung für den Festkörperlaser ist das in ihm enthaltene laseraktive Medium, in dem die Laserstrahlung generiert wird. Im Falle des Festkörperlasers wird das laseraktive Medium von einem Kristall gebildet, welcher nach verschiedenen Methoden bis zur Populationsinversion angeregt werden kann.
Zum einen ist als Stand der Technik bei der Anregung des Laserkristalls das optische Pumpen mit einer Blitzlampe und zum anderen das optische Pumpen mit einem anderen Lasersystem bekannt.
Bei der Anregung mittels einer Blitzlampe liegt ein Teil des von der Blitzlampe emittierten Spektrums im Bereich der zur Laseranregung notwendigen Absorptionsbande des Laserkristalls. Die Anregung des Kristalls geschieht dabei durch eine transversale Anordnung, d.h. Laserkristall und Blitzlampe liegen parallel nebeneinander. Die von der Blitzlampe abgestrahlte unerwünschte Wärmeleistung macht eine Kühlung des Laserkristalls unumgänglich.
Die Anregung mittels eines anderen Lasersystems kann in verschiedenen Anordnungsmöglichkeiten erfolgen:
1: Der Laser, der zum Pumpen des Kristalls eingesetzt wird, strahlt in der longitudinalen Konfiguration, das heißt entlang der Längsachse des Kritalls.
2: Ein Array von Lasersystemen wird in der transversalen Konfiguration, das heißt, transversal zum Kristall, angeordnet.

Der Vorteil der Anregung des Lasermediums mittels eines anderen Lasers ist die schmalbandige Anregung des Laserübergangs durch die excited state absorption (ESC). Bei diesem Prozess geht im Vergleich zur breitbandigen Anregung mit einer Blitzlampe nur minimal Anregungsenergie verloren. Nachteilig ist jedoch, dass die Pumpenergie, zum Beispiel bei der longitudinalen Anregung, nicht gleichmäßig im Kristall verteilt wird.

EP 867 151 A2 beschreibt ein derartiges Gerät entsprechend dem Oberbegriff des Anspruchs 1.

Ein weiteres Verfahren zur Erzeugung der Populationsinversion ist das sogenannte diffuse Pumpen. Dieses Verfahren ist in der deutschen Patentanmeldung 100 13 371.1 beschrieben. Die bei diesem Verfahren verwendete Pumpkonfiguration kann weder als transversal noch als longitudinal beschrieben werden. Vielmehr wird die Pumpstrahlung für den Kristall über spezielle Lichttransmissionssysteme in die Pumpkammer eingekoppelt. Durch Mehrfachreflektion an der Wandinnenfläche der Pumpkammer erfolgt die homogene Beleuchtung des Laserkristalls. Die Pumplichtquelle kann dabei von einem oder mehreren Lasern gebildet werden.

Bei den Diodenlasern werden Halbleiterkristalle als aktive Medien verwendet, die bei Anregung eine kohärente Strahlung im sichtbaren und im nahen infraroten Spektralbereich emittieren. In Halbleitern sind die Energiezustände der Elektronen nicht scharf wie bei freien Atomen, sondern durch breite Bänder gegeben. Den Grundzustand bildet das Valenzband, den angeregten Zustand das Leitungsband. Die Anregung erfolgt üblicherweise am sogenannten pn- Übergang nach Anlegen einer äußeren Spannung. Die Elektronen werden vom Valenzband in das Leitungsband befördert, was zur Besetzungsinversion führt. Bei einer darauf folgenden stimulierten Emission kehren sie in das Valenzband zurück und senden dabei Licht aus. Die Emissionswellenlänge hängt vom energetischen Abstand zwischen Valenz- und Leitungsband ab, wobei sich der Bandabstand aus der Auswahl geeigneter Halbleiterverbindungen ergibt. Dies sind in der Regel die Elemente der II. bis IV. Gruppe des Periodensystems und/oder Mischkristalle aus der III. bis V.-Gruppe, die von besonderer Bedeutung sind.

Es ist die Aufgabe der Erfindung, ein in der Medizin anwendbares Lasergerät zu schaffen, welches zum einen eine größere Vielzahl von Einsatzmöglichkeiten bietet und sich zum anderen durch eine kleine kompakte Bauweise auszeichnet. Das Gerät soll gut transportabel und als Modulbaustein in verschiedene Geräte integrierbar sein.

Ausgehend vom Oberbegriff des Anspruchs 1 wird die Aufgabe gelöst durch die im kennzeichnenden Teil des Anspruchs 1 angegebenen Merkmale.

Mit dem erfindungsgemäßen medizinischen Behandlungsgerät, beziehungsweise dem medizinischen Laserbehandlungsgerät ist es nunmehr möglich, mit nur einem Gerät eine Vielzahl von medizinischen Behandlungen durchzuführen, die unterschiedliche Anforderungen an die Wellenlänge der Laserstrahlung stellen. Je nach Behandlungsart kann mit nur einem Gerät die gewünschte Wellenlänge erzeugt werden.

Vorteilhafte Weiterbildungen der Erfindung sind in den Unteransprüchen angegeben.

Die Zeichnungen zeigen die Funktionsweise und den Aufbau des erfindungsgemäßen medizinischen Laserbehandlungsgerätes in schematischer Form.

Es zeigt :
- Fig.1:: Eine schematische Darstellung der Funktionsweise des erfindungsgemäßen medizinischen Laserbehandlungsgerätes.
- Fig.2:: Eine beispielhafte Ausführungsform der Erfindung.
- Fig.3:: Eine Übersicht des medizinischen Laserbehandlungsgerätes.

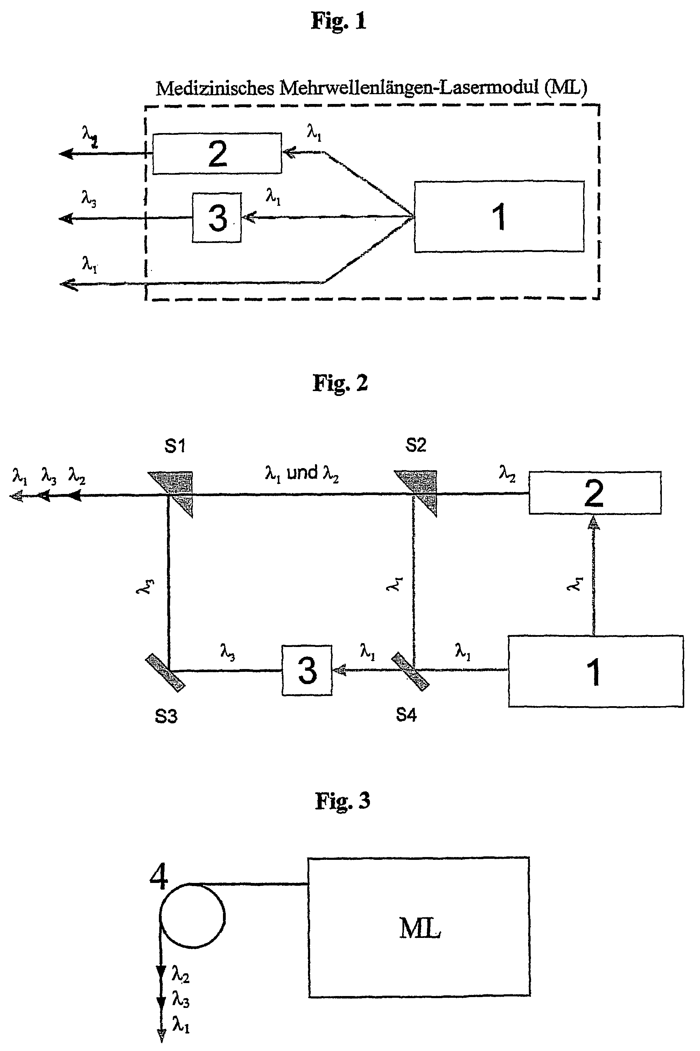

In dem in Figur 1 dargestellten Blockdiagramm bezeichnet das Bezugszeichen 1 einen Diodenlaser, beziehungsweise ein Diodenlaserarray. Die Ausführungsformen beziehen sich sowohl auf einen einzelnen Diodenlaser als auch auf ein Diodenlaserarray. Bei einem Diodenlaserarray können sowohl die Strahlen vereint werden, als auch einzeln weitergeführt werden, beispielsweise durch Einleitung in voneinander getrennte Lichtleitfasern. Das Festkörperlasermodul wird mit dem Bezugszeichen 2 und eine nichtlineare Verdopplereinheit mit 3 bezeichnet. Der Buchstabe λ₂ bezeichnet die Emissionswellenlänge des Festkörperlasers 2, λ₃ die Emissionswellenlänge der nichtlinearen Verdopplereinheit 3 und λ₁ die Emissionswellenlänge des Diodenlasers 1.

Es ist besonders zweckmäßig, dass das medizinische Laserbehandlungsmodul so ausgestattet ist, dass es ein Lichttransmissionssystem mit einem Flüssigkeitslichtleiter enthält. Vorzugsweise dient der Flüssigkeitslichtleiter zur Übertragung von verschiedenen Wellenlängen über einen sehr großen Spektralbereich, wie dies in der Ausführungsform des Mehrwellenlasermoduls gegeben ist.

Die in Figur 2 dargestellte Versuchsanordnung zeigt einen beispielhaften Aufbau zur Strahlenüberlagerung. In ihr erzeugt der Diodenlaser 1 die Emissionswellenlänge λ₁, welche in den Festkörperlaser 2 eingekoppelt wird, der seinerseits die Wellenlänge λ₂ emittiert. Die vom Festkörperlaser 2 emittierte Laserstrahlung der Wellenlänge λ₂ durchläuft die teildurchlässigen Spiegel S2 und S1, bevor er das Lasermodul verlässt. Ein anderer Teilstrahl der Wellenlänge λ₁ des Diodenlasers 1 trifft auf den Strahlenteiler S4, wird auf dem teildurchlässigen Spiegel S2 reflektiert und verlässt vorzugsweise strahlüberlagernd mit dem Strahl der Wellenlänge λ₂ nach seinem Strahlenweg durch den teildurchlässigen Spiegel S1, ebenfalls das Lasermodul. Der andere am Strahlenteiler S4 erzeugte Teilstrahl mit der Wellenlänge λ₁ wird in die nichtlineare Verdopplereinheit eingekoppelt und dort in die Wellenlänge λ₃ transformiert. Der Strahl mit der Wellenlänge λ₃ wird am Spiegel 3 vollständig reflektiert und verlässt durch Reflektion am teildurchlässigen Spiegel S1 ebenfalls, vorzugsweise strahlüberlagernd mit λ₁ und λ₂, das Lasermodul.

In Figur 3 ist dargestellt, wie die überlagerten Strahlen der Wellenlängen λ₁, λ₂ und λ₃ eines Lasermoduls ML über ein Lichttransmissionssystem 4 in einer überlagerten Achse zum Zielort geleitet werden.

Im Folgenden soll das erfindungsgemäße medizinische Laserbehandlungsmodul in verschiedenen Ausführungsformen beschrieben werden.

Das medizinische Laserbehandlungsmodul ist erfindungsgemäß derart ausgestaltet, dass es eine Laserstrahlungsquelle 1 für die Erzeugung einer Grundwellenlänge λ₁ besitzt und dass es weiterhin mindestens ein Mittel 2,3 zur Erzeugung von Laserstrahlung einer weiteren Wellenlänge λ₂, λ₃ und wenigstens ein Mittel zum wahlweisen Einkoppeln der Laserstrahlung der Grundwellenlänge λ₁ in das Mittel 2,3 zur Erzeugung der Wellenlänge λ₂, λ₃ besitzt. Vorzugsweise sind zwei Mittel 2,3 zur Erzeugung von Laserstrahlung einer weiteren Wellenlänge λ₂, λ₃ vorhanden.

Es eignen sich eine Vielzahl von Mitteln zur Wellenlängeneinkopplung der Grundwellenlängen λ₁ in die Mittel 2, 3. Die Darstellung, bei der das Mittel 2 zur Erzeugung von Laserstrahlung der Wellenlänge λ₂ ein Festkörperkristall ist, stellt eine bevorzugte Ausführungsform dar. Bei dem Mittel zur Erzeugung von Laserstrahlung der Wellenlänge λ₂ kann es sich jedoch auch um ein anderes Mittel handeln. Vorzugsweise handelt es sich bei dem weiteren Mittel zur Erzeugung einer weiteren Wellenlänge um eine Verdopplereinheit, welche Laserstrahlung der Wellenlänge λ₃ erzeugt.

Zur Einkopplung von Laserstrahlung in die Mittel 2 und 3 dienen geeignete Lichttransmissionssysteme wie beispielsweise Flüssigkeitslichtleiter oder Festkörperfasern, insbesondere Glasfasern.

Eine wahlweise Einkopplung der Laserstrahlung erfolgt vorzugsweise durch geeignete Umlenksysteme, die sich aus geeigneten Mitteln wie Spiegelsystemen, Strahlteilern, dichroitischen Spiegeln oder einschwenkbaren Spiegeln bestehen. Die Mittel zum Einkoppeln der Laserstrahlung der Grundwellenlänge λ₁ können ferner geeignete Linsenelemente umfassen.

Die Auskopplung von Laserstrahlung aus den Mitteln 1, 2 und 3 zur Erzeugung von Laserstrahlung der Wellenlängen λ₁, λ₂ und λ₃ weist vorzugsweise die zuvor anhand der Einkopplung von Laserstrahlung erläuterten Mittel auf.

Vorzugsweise werden auch zur Auskopplung von Laserstrahlung aus den Mitteln 1, 2 und 3 geeignete Lichttransmissionssysteme vorzugsweise unter Einsatz von Flüssigkeitslichtleitern oder Festkörperfasern eingesetzt.

Als bevorzugte Ablenksysteme werden geeignete Spiegelsysteme, beispielsweise Strahlteiler, dichroitische Spiegel oder einschwenkbare Spiegel eingesetzt.

Zur Auskopplung dienen vorzugsweise Prismen- oder Linsenelemente.

In einer bevorzugten Ausführungsform der Erfindung generiert das Mittel 1 zur Erzeugung der Laserstrahlung der Wellenlänge λ₁ eine kürzere Wellenlänge als das Mittel 2 zur Erzeugung der Laserstrahlung λ₂ und eine längere Wellenlänge als das Mittel 3 zur Erzeugung der Laserstrahlung der Wellenlänge λ₃.

Vorzugsweise wird dies dadurch erreicht, dass als Mittel 1 zur Erzeugung der Wellenlänge λ₁ ein Diodenlaser, als Mittel 2 zur Erzeugung der Wellenlänge λ₂ ein Festkörperlaser und als Mittel 3 zur Erzeugung der Wellenlänge λ₃ ein nichtlinearer Frequenzverdoppler eingesetzt werden.

Der Einsatz des Diodenlasers als Mittel zur Erzeugung der Wellenlänge λ₁ hat zum einen den Vorteil, dass dieser ein sehr kleines Bauteil der erfindungsgemäßen Vorrichtung darstellt, was für den Einsatz als medizinisches Behandlungsgerät, insbesondere für ein mobiles Behandlungsgerät von großem Vorteil ist. Die von Diodenlasern generierten Wellenlängen λ₁ sind in der medizinischen Behandlung auch direkt anwendbar. So kann Licht von Diodenlasern in einem Wellenlängenbereich von 900 nm bis 1000 nm vorzugsweise zur Behandlung im Bereich der Parodontologie, der Endodontie, und der Chirurgie eingesetzt werden. Dies ist insbesondere in der Zahnmedizin von besonderer Bedeutung. Gemäß den gewünschten Wellenlängen kann der Diodenlaser 1 vorzugsweise ein aktives Medium aus der Gruppe Gallium-Arsenid (GaAs), Indium-Gallium-Arsenid (InGaAs), Gallium-Aluminium-Arsenid (GaAlAs), Indium-Gallium-Aluminium-Arsenid (InGaAlAs) und Indium-Gallium-Arsenid-Phosphit (InGaAsP) besitzen. Die Auswahl ist jedoch nicht auf diese Gruppe beschränkt. Statt dessen kann jedes aktive Medium zum Einsatz kommen, das für eine medizinische Behandlung geeignet ist und/oder zur Anregung eines weiteren Lasers dienen kann, welcher seinerseits geeignet ist, eine medizinisch anwendbare Wellenlänge λ₂, λ₃ zu erzeugen. An Stelle eines einzelnen Diodenlasers 1 kann auch ein Diodenlaserarray treten.

In einer weiteren bevorzugten Ausführungsform kann als Mittel 2 zur Erzeugung der Wellenlänge λ₂ eine Laserstrahlungsquelle eingesetzt werden, welche Laserstrahlung einer Wellenlänge λ₂ in einem Bereich von 1,5 µm bis 3 µm erzeugen kann. Vorzugsweise kann ein Festkörperlaser 2 eingesetzt werden, der ein aktives Medium besitzt, welches befähigt ist, Laserstrahlung der Wellenlänge λ₂ in einem Wellenlängenbereich zwischen 1,5 µm und 3 µm zu erzeugen. Als aktives Medium können beispielsweise Kristalle aus der Gruppe Nd:YAG, Nd:YLF, Ho:YAG, Er:YAG, ErCr:YSGG, Er:GGG, Er:YSGG, Er:YLF, CrTmEr:YAG oder mit anderen seltenen Erden dotierte Kristalle eingesetzt werden. Jedoch sind die verwendbaren Kristalle nicht auf diese Gruppe beschränkt, vielmehr kann jeder Kristall eingesetzt werden, welcher fähig ist, Laserstrahlung einer Wellenlänge zu erzeugen, welche für die medizinische Behandlung geeignet ist. Die Wahl des Diodenlasers 1, beziehungsweise des Diodenlaserarrays zur Erzeugung der Grundwellenänge λ₁ des erfindungsgemäßen medizinischen Laserbehandlungsgerätes hängt davon ab, welcher Festkörperlaserkristall zur Erzeugung der Laserstrahlung der Wellenlänge λ₂ ausgewählt wird. Die beispielhaft für den Festkörperlaser 2 aufgezählten Kristalle eignen sich für die Verwendung mit den für die beispielhaft für den Diodenlaser 1 aufgezählten Diodenlaserkristallen. Die Laserstrahlung der Festkörperlaser 2 kann beispielsweise bei der Kavitätenpräparation, Parodontologie, der Endodontie oder der Bearbeitung von Kunststoffen eingesetzt werden.

Beim Einsatz von Festkörperlasern 2 als Mittel zur Erzeugung der Wellenlänge λ₂ wird der Festkörperlaser 2 durch das Mittel zur Erzeugung der Wellenlänge λ₁ optisch gepumpt. Hierzu kommen zwar alle denkbaren Pumpmechanismen in Frage, besonders bevorzugt ist jedoch das Verfahren des diffusen Pumpens, insbesondere das Verfahren, welches in der deutschen Patentanmeldung DE 100 13 371 beschrieben ist. Beim diffusen Pumpen befindet sich das aktive Medium des Festkörperlasers 2 in einer Kavität, welche an ihren beiden Enden, vorzugsweise über die Breitseiten und über den gesamten Umfang verspiegelt ist. Bevorzugt ist der Innenraum der Kavität mit einer Flüssigkeit gefüllt, welche sich ebenfalls in einer Leitung befindet, die das Licht der Wellenlänge λ₁ in den Festkörperlaser 2 einkoppelt. Hierdurch wird der Innenraum der Kavität des Festkörperlasers 2 homogen mit Licht ausgeleuchtet. Als Flüssigkeit können beispielsweise wässrige Lösungen, Silikonöle und/oder andere geeignete Flüssigkeiten in Frage kommen. In einer weiteren bevorzugten Ausführungsform der Erfindung wird die zum Einkoppeln des Lichtes der Wellenlänge λ₁ in den Festkörperlaser 2 verwendete Flüssigkeit in einem Kreislauf geführt, welcher mit einem Kühlaggregat ausgestattet ist. Somit ist eine Kühlung des Innenraumes der Kavität möglich.

Als Mittel 3 zur Erzeugung der Wellenlänge λ₃ wird bevorzugt eine Laserstrahlungsquelle 3 eingesetzt, welche befähigt ist, Laserstrahlung einer Wellenlänge λ₃ in einem Wellenlängenbereich von 450 nm bis 500 nm zu erzeugen. Bevorzugt werden hierzu nichtlineare Frequenzverdoppler 3 eingesetzt, in welche fakultativ das Licht der Wellenlänge λ₁ eingekoppelt wird, um den Frequenzverdopplerkristall zu pumpen. Als besonders geeignet haben sich nichtlineare Frequenzverdoppler 3 mit Verdopplerkristallen aus der Gruppe KTP-, KDP-, LiNbO₃-, KNbO₃-, LiTaO₃ und LBO -Kristallen herausgestellt. Mit einer zusätzlichen periodischen Polung kann das Leistungsspektrum einiger Kristalle, wie zum Beispiel KTP oder LiTaO₃ erheblich gesteigert werden. Diese Kristalle können in Abhängigkeit von der Pumpwellenlänge Laserstrahlung erzeugen, welche beispielsweise in der Chirurgie, in der Endodontie und bei der Polymerisation von Kunststoffen eingesetzt werden kann. Die Auswahl soll jedoch nicht auf die Beispiele beschränkt sein, vielmehr kann jeder Kristall zum Einsatz kommen, der Laserstrahlung einer Wellenlänge erzeugen kann, welche für insbesondere medizinische Anwendungen geeignet ist. In einer bevorzugten Ausführungsform ist der nichtlineare Frequenzverdoppler in einen Resonator eingebettet, so dass eine zusätzliche Verstärkung seines Emissionsspektrums möglich ist.

Erfindungsgemäß sind die Mittel zur Erzeugung der Wellenlängen λ₁, λ₂ und λ₃ derart in ein Modul eingebracht, dass mindestens eine Komponente aus der Gruppe der Wellenlängen λ₁, λ₂ und λ₃ des Lasermoduls durch ein Lichttransmissionssystem 4 an den Behandlungsort geführt werden kann. Als Lichttransmissionssystem 4 kommen beispielsweise ein Glasfaserkabel oder eine mit Flüssigkeit gefüllte beliebige Hohlstruktur (Lumen) in Betracht, welche das Licht an eine Austrittsstelle weiterleiten, die die medizinische Behandlung entweder durch manuelle Handhabung oder rein mechanisch gesteuert ausführt. In dieses Lichttransmissionssystem 4 werden je nach Behandlungsbedarf die gewünschten Wellenlängen eingekoppelt. Die Einkopplung des Lichtes der verschiedenen Wellenlängen kann beispielsweise durch Anordungen von Strahlteilern S4, wie halbdurchlässigen Spiegeln S1,S2 und Spiegeln S3 erfolgen.

Eine in Figur 2 dargestellte Anordnung von Modulbestandteilen zeigt eine beispielhafte Ausführungsform. In ihr emittiert ein Diodenlaser 1 Licht einer Wellenlänge λ₁, welches über den Strahlteiler S4 und den halbdurchlässigen Spiegel S2, S1 zum einen direkt und zum anderen über den Strahlteiler S4 einem nichtlinearen Frequenzverdoppler 3 und dann nach Umwandlung in Licht der Wellenlänge λ₃ über den Spiegel S3 und den halbdurchlässigen Spiegel S1 einem Lichttransmissionssystem 4 zugeführt wird. Weiterhin wird ein Teil des Lichtes der Wellenlänge λ₁ direkt aus dem Diodenlaser 1 ausgekoppelt und pumpt einen Festkörperlaser 2, dessen Licht der Wellenlänge λ₂ die halbdurchlässigen Spiegel S2 und S1 durchläuft und dem Lichttransmissionssystem 4 zugeführt wird. Aufgrund der Tatsache, dass die Wellenlängen λ₂ und λ₃ in Abhängigkeit der Wellenlänge λ₁ generiert werden, sind die Ausgangsleistungen von λ₂ und λ₃ direkt an die Ausgangsleitung von λ₁ gekoppelt. Daher erfolgt die Einstellung der Laserleistung bei den Wellenlängen λ₁, λ₂ und λ₃ anhand der Leistung der Grundwellenlänge λ₁, das heißt im vorliegenden Beispiel anhand der Leitungsregelung des Diodenlasers 1 oder des Diodenlaserarrays 1.

### Anwendungsbeispiele:

Speziell in der Zahnmedizin ist bei einer bevorzugten Ausführungsform der Erfindung vorgesehen, das Mehrwellenlängenlasermodul in den Bereichen der Kavitätenpräparation, Parodontologie, Chirurgie, Endodontie und zur Bearbeitung und Polymerisation von Kunststoffen einzusetzen. Hierzu können folgende Wellenlängen verwendet werden:
Kavitätenpräparation: 2 µm bis 3 µm, (λ₂)
Parodontologie: 900 nm bis 1000 nm sowie 2 µm bis 3 µm, (λ₁ und λ₂).
Chirurgie: 450 nm bis 500 nm, 900 nm bis 1000 nm, (λ₁ und λ₃).
Endodontie: 450 nm bis 500 nm, 900 nm bis 1000 nm, 2 µm bis 3 µm, (λ₁, λ₂ und λ₃).
Bearbeitung und Polymerisation von Kunststoffen: 2 µm bis 3 µm, : 450 nm bis 500 nm, (λ₂ und λ₃).

In Abhängigkeit des Einsatzgebietes in der Medizin ist in einer bevorzugten Ausführungsform der Erfindung für die Zahnmedizin vorgesehen, das Emissionsspektrum wie folgt auszuwählen:
Grundwellenlänge λ₁ des Diodenlasers, beziehungsweise des Diodenlaserarrays 1 im Bereich von 900 nm bis 1000 nm,
Wellenlänge λ₂ des Festkörperlasers 2 von 1,5 µm bis 3 µm,
Wellenlänge λ₃ der Verdopplereinheit 3 von 450 nm bis 500 nm.

Weiterhin kann das erfindungsgemäße medizinische Laserbehandlungsmodul in verschiedenen Bereichen der Humanmedizin, wie beispielsweise Dermatologie, Ophthalmologie oder Zahnmedizin sowie in der Tiermedizin eingesetzt werden.

Das erfindungsgemässe medizinische Laserbehandlungsmodul ermöglicht die Erzeugung mehrerer Laserwellenlängen innerhalb eines sehr kleinen und kompakten Aufbaus, der als solitäres Gerät, das heißt als komplette selbständige Einheit, als modularer Baustein (Wellenlängen ausgewählt) oder als integrierbares Modul in der Praxis eingesetzt werden kann. Die Auswahl der Laserwellenlängen λ₁, λ₂ und λ₃ und somit die Auswahl der Mittel zur Erzeugung der Laserstrahlung richten sich nach dem Einsatzgebiet in der Medizin. Bei geeigneter Strahlanordnung können diese einzeln, in Kombination oder in vollständiger Überlagerung aus dem erfindungsgemäßen medizinischen Laserbehandlungsmodul mittels eines geeigneten Lichttransmissionssystems 4 herausgeführt werden, wie es in Figur 3 dargestellt ist.

Bei einer bevorzugten Ausführungsform des erfindungsgemäßen medizinischen Laserbehandlungsmoduls ist vorgesehen, dieses als solitäres Gerät aufzubauen. Darin sind alle Modulkomponenten, wie das Diodenlaser- oder das Diodenlaserarray-Modul 1 zur Generierung der Grundwellenlänge λ₁, das Festkörperlasermodul 2 zur Erzeugung der niederfrequenten Laserstrahlung λ₂ und die Verdopplereinheit 3 zur Erzeugung der Laserwellenlänge λ₃ enthalten. Weiterhin kann bei geeigneter Strahlanordnung mindestens eine Komponente aus der Gruppe der Wellenlängen λ₁, λ₂ und λ₃ einzeln oder in vollständiger Überlagerung aus dem erfindungsgemäßen medizinischen Laserbehandlungsgerät herausgeführt werden. Der Strahltransport erfolgt aus dem Gerät über ein oder mehrere geeignete Lichttransmissionssysteme 4 zum Anwendungsort.

In einer weiteren bevorzugten Ausführungsform ist vorgesehen, das erfindungsgemäße Gerät als modularen Baustein aufzubauen. Hierbei ist eine Kombination einzelner Modulkomponenten möglich, wie z.B. das Diodenlaser- bzw. das Diodenlaserarray-Modul 1 zur Generierung der Grundwellenlänge λ₁ und das Festkörperlasermodul 2 zur Erzeugung der niederfrequenten Laserstrahlen der Wellenlänge λ₂ oder das Diodenlaser- bzw. das Diodenlaserarray-Modul 1 zur Generierung der Grundwellenlänge λ₁ und die Verdopplereinheit 3 zur Erzeugung der Wellenlänge λ₃. Weiterhin können bei geeigneter Strahlanordnung die Laserwellenlängen λ₃, λ₁ bzw. λ₂, λ₁ einzeln oder in vollständiger Überlagerung, das heißt λ₃ plus λ₁ bzw. λ₂ plus λ₁, durch ein Lichttransmissionssystem 4 aus einem Baustein herausgeführt werden.

In einer weiteren bevorzugten Ausführungsform der Erfindung kann das medizinische Laserbehandlungsmodul als integrierbares Modul beispielsweise in eine zahnärztliche Behandlungseinheit integriert werden. Dies kann wiederum als solitäres System oder als modularer Baustein erfolgen. Bezüglich der Wellenlängenkombination können alle beschriebenen Variationen des solitären Systems bzw. des modularen Bausteins in Betracht gezogen werden.

### Bezugszeichenliste:

- 1.: Diodenlaser bzw. Diodenlaserarray mit λ₁: Grundwellenlänge
- 2.: Festkörperlasermodul mit λ₂: niederfrequentes Emissionsspektrum
- 3.: Verdopplereinheit mit λ₃: höherfrequentes Emissionsspektrum
- 4.: Lichttransmissionssystem

- S 1: Umlenkprisma, Strahlteiler oder Spiegel
- S2: Umlenkprisma, Strahlteiler oder Spiegel
- S3: Umlenkprisma, Strahlteiler oder Spiegel
- S4: Umlenkprisma, Strahlteiler oder Spiegel

## Patentansprüche

1. Medizinisches Laserbehandlungsmodul, umfassend wenigstens eine erste Laserstrahlungsquelle (1) für die Erzeugung einer Grundwellenlänge (λ₁) und wenigstens ein Mittel (2,3) zur Erzeugung von Laserstrahlung einer weiterer Wellenlänge (λ₂, λ₃) sowie einem Mittel zum Einkoppeln der Laserstrahlung der Grundwellenlänge (λ₁) in das Mittel zur Erzeugung der weiteren Wellenlängen (λ₂, λ₃), **dadurch gekennzeichnet, dass** in einem Betriebszustand sowohl die Laserstrahlung der Grundwellenlänge (λ₁) als auch die Laserstrahlung von mindestens einer der weiteren Wellenlängen (λ₂, λ₃) aus dem medizinischen Mehrwellenlängen-Lasermodul auskoppelbar sind, dass die Wellenlängen λ₂ und λ₃ in Abhängigkeit der Wellenlänge λ₁ generiert werden, dass die erste Laserstrahlungsquelle (1) ein Diodenlaser, das Mittel (2) zur Erzeugung der Wellenlänge λ₂ ein Festkörperlaser und das Mittel (3) zur Erzeugung der Wellenlänge λ₃ ein nichtlinearer Frequenzverdoppler ist, und dass durch eine geeignete Strahlanordnung mindestens eine Komponente aus der Gruppe der Wellenlängen λ₁, λ₂ und λ₃ einzeln oder in vollständiger Überlagerung aus dem medizinischen Laserbehandlungsmodul mittels eines Lichttransmissionssystems herausgeführt werden kann.

2. Medizinisches Laserbehandlungsmodul nach Anspruch 1, **dadurch gekennzeichnet, dass** der Festkörperlaser zur Erzeugung der Laserstrahlung der Wellenlänge (λ₂) eine längere Wellenlänge und der nichtlineare Frequenzverdoppler zur Erzeugung der Laserstrahlung der Wellenlänge (λ₃) eine kürzere Wellenlänge als (λ₁) erzeugt.

3. Medizinisches Laserbehandlungsmodul nach einem oder beiden der Ansprüche 1 oder 2, **dadurch gekennzeichnet, dass** das aktive Medium des Diodenlasers eine Komponente ist aus der Gruppe von Gallium-Arsenid (GaAs), Indium-Gallium-Arsenid (InGaAs), Gallium-Aluminium-Arsenid (GaAlAs), Indium-Gallium-Aluminium-Arsenid (InGaAlAs) oder Indium-Gallium-Arsenid-Phosphit (InGaAsP).

4. Medizinisches Laserbehandlungsmodul nach einem oder mehreren der vorangegangenen Ansprüche, **dadurch gekennzeichnet, dass** der Diodenlaser fähig ist, Licht einer Wellenlänge in einem Wellenlängenbereich λ₁ von 900nm bis 1000nm zu erzeugen.

5. Medizinisches Laserbehandlungsmodul nach einem oder mehreren der vorangegangenen Ansprüche, **dadurch gekennzeichnet, dass** mehrere Diodenlaser in einem Diodenlaserarray angeordnet sind.

6. Medizinisches Laserbehandlungsmodul nach einem oder mehreren der vorangegangenen Ansprüche, **dadurch gekennzeichnet, dass** der Festkörperlaser einen laseraktiven Kristall aus der Gruppe Nd:YAG, Nd:YLF, Ho:YAG, Er:YAG, ErCr:YSGG, Er:GGG, Er:YSGG, Er:YLF, CrTmEr:YAG oder einen mit anderen seltenen Erden dotierten Kristall besitzt.

7. Medizinisches Laserbehandlungsmodul nach einem oder mehreren der vorangegangenen Ansprüche, **dadurch gekennzeichnet, dass** der Festkörperlaser fähig ist, eine Wellenlänge in einem Bereich zwischen 1,5 µm und 3 µm zu erzeugen.

8. Medizinisches Laserbehandlungsmodul nach einem oder beiden der Ansprüche 6 oder 7, **dadurch gekennzeichnet, dass** der Kristall des Festkörperlasers in einer Kavität eingelagert ist, welche ein diffuses Pumpen ermöglicht.

9. Medizinisches Laserbehandlungsmodul nach Anspruch 8, **dadurch gekennzeichnet, dass** die Kavität des Festkörperlasers über eine Flüssigkeitszuleitung mit der Laserstrahlungsquelle (1) derart verbunden ist, class das Licht der Wellenlänge (λ₁) über die Flüssigkeit in die Kavität zum Pumpen des Kristalls eingekoppelt werden kann.

10. Medizinisches Laserbehandlungsmodul nach Anspruch 9, **dadurch gekennzeichnet, dass** die Flüssigkeitszuleitung als Kreislauf ausgebildet ist, welcher ein Kühlaggregat durchläuft.

11. Medizinisches Laserbehandlungsmodul nach einem oder beiden der Ansprüche 9 oder 10, **dadurch gekennzeichnet, dass** die Flüssigkeitszuleitung und die Kavität mit wässrigen Lösungen, Silikonöl und/oder anderen geeigneten Flüssigkeiten gefüllt sind.

12. Medizinisches Laserbehandlungsmodul nach einem oder mehreren der vorangegangenen Ansprüche, **dadurch gekennzeichnet, dass** der nichtlineare Frequenzverdoppler einen Verdopplerkristall aus der Gruppe KTP, KDP, LiNbO₃, KNbO₃, LiTaO₃ und LBO besitzt.

13. Medizinisches Laserbehandlungsmodul nach einem oder mehreren der vorangegangenen Ansprüche, **dadurch gekennzeichnet, dass** der nichtlineare Frequenzverdoppler Wellenlängen im Bereich von (λ₃) zwischen 450 nm und 500 nm erzeugt.

14. Medizinisches Laserbehandlungsmodul nach einem oder mehreren der vorangegangenen Ansprüche, **dadurch gekennzeichnet, dass** der Frequenzverdoppler zur Verstärkung seiner Emissionswellenlänge (λ₃) mit einem Resonator ausgestattet ist.

15. Medizinisches Laserbehandlungsmodul nach einem oder mehreren der Ansprüche 1 bis 14, **dadurch gekennzeichnet, dass** es ein Lichttransmissionssystem mit einem Flüssigkeitslichtleiter enthält.

16. Medizinisches Laserbehandlungsmodul nach einem oder mehreren der Ansprüche 1 bis 15, **dadurch gekennzeichnet, dass** es ein in ein medizinisches Behandlungsgerät integrierbares Modul ist.

17. Ärztliches Behandlungsinstrument, **dadurch gekennzeichnet, dass** es über ein medizinisches Laserbehandlungsmodul nach einem oder mehreren der Ansprüche 1 bis 16 verfügt.

18. Ärztliches Behandlungsinstrument nach Anspruch 17, **dadurch gekennzeichnet, dass** es ein zahnärztliches Behandlungsinstrument ist.

## Claims

1. A medical laser treatment module comprising at least a first source of laser radiation (1) for generating a fundamental wavelength (λ₁) and at least one means (2, 3) for generating laser radiation having an additional wavelength (λ₂, λ₃) as well as a means for coupling the laser radiation having the fundamental wavelength (λ₁) into the means for generating the additional wavelengths (λ₂, λ₃), **characterized in that** in one operating state, the laser radiation having the fundamental wavelength (λ₁) as well as the laser radiation having at least one of the additional wavelengths (λ₂, λ₃) can be decoupled out of the medical multiple wavelength laser module, whereby the wavelengths (λ₂ and λ₃) are generated as a function of the wavelength (λ₁), whereby the first source of laser radiation (1) is a diode laser, the means (2) for generating the wavelength λ₂ is a solid state laser and the means (3) for generating the wavelength λ₃ is a non-linear frequency doubler, and whereby, with a suitable beam arrangement, at least one component from the group consisting of wavelengths λ₁, λ₂ and λ₃ can be conveyed out of the medical laser treatment module either individually or in complete superimposition by means of a light transmission system.

2. The medical laser treatment module according to Claim 1, **characterized in that** the solid state laser for generating the laser radiation having the wavelength (λ₂) generates a longer wavelength than (λ₁) and the non-linear frequency doubler for generating the laser radiation having the wavelength (λ₃) generates a shorter wavelength than (λ₁).

3. The medical laser treatment module according to one or both of Claims 1 or 2, **characterized in that** the active medium of the diode laser is a component from the group consisting of gallium-arsenide (GaAs), indium-gallium-arsenide (InGaAs), gallium-aluminium-arsenide (GaAlAs), indium-gallium-aluminium-arsenide (InGaAlAs) or indium-gallium-arsenide-phosphite (InGaAsP).

4. The medical laser treatment module according to one or more of the preceding claims, **characterized in that** the diode laser is capable of generating light having a wavelength in the range λ₁ from 900 nm to 1000 nm.

5. The medical laser treatment module according to one or more of the preceding claims, **characterized in that** several diode lasers are arranged in a diode laser array.

6. The medical laser treatment module according to one or more of the preceding claims, **characterized in that** the solid state laser has a laser-active crystal from the group consisting of Nd:YAG, Nd:YLF, Ho:YAG, Er:YAG, ErCr:YSGG, Er:GGG, Er:YSGG, Er:YLF, CrTmEr:YAG or a crystal doped with other rare earths.

7. The medical laser treatment module according to one or more of the preceding claims, **characterized in that** the solid state laser is capable of generating a wavelength in the range from 1.5 µm to 3 µm.

8. The medical laser treatment module according to one or both of Claims 6 or 7, **characterized in that** the crystal of the solid state laser is embedded in a cavity that allows diffuse pumping.

9. The medical laser treatment module according to Claim 8, **characterized in that** the cavity of the solid state laser is connected to the source of laser radiation (1) via a liquid feed line in such a way that the light having the wavelength (λ₁) can be coupled into the cavity via the liquid in order to pump the crystal.

10. The medical laser treatment module according to Claim 9, **characterized in that** the liquid feed line is configured as a circulation system that passes through a cooling aggregate.

11. The medical laser treatment module according to one or both of Claims 9 or 10, **characterized in that** the liquid feed line and the cavity are filled with aqueous solutions, silicone oil and/or other suitable liquids.

12. The medical laser treatment module according to one or more of the preceding claims, **characterized in that** the non-linear frequency doubler has a doubler crystal from the group consisting of KTP, KDP, LiNbO₃, KNbO₃, LiTaO₃ and LBO.

13. The medical laser treatment module according to one or more of the preceding claims, **characterized in that** the non-linear frequency doubler generates wavelengths in the range of (λ₃) from 450 nm to 500 nm.

14. The medical laser treatment module according to one or more of the preceding claims, **characterized in that** the frequency doubler is equipped with a resonator in order to amplify its emission wavelength (λ₃).

15. The medical laser treatment module according to one or more of Claims 1 to 14, **characterized in that** it contains a light transmission system with a liquid light conductor.

16. The medical laser treatment module according to one or more of Claims 1 to 15, **characterized in that** it is a module that can be integrated into a medical treatment device.

17. A medical treatment instrument, **characterized in that** it has a medical laser treatment module according to one or more of Claims 1 to 16.

18. The medical treatment instrument according to Claim 17, **characterized in that** it is a dental treatment instrument.

## Revendications

1. Module de traitement à laser médical comprenant au moins une première source de rayonnement laser (1) générant une longueur d'ondes de base (λ₁) et au moins un moyen (2, 3) pour générer un rayonnement laser d'une autre longueur d'ondes (λ₂, λ₃) ainsi qu'un moyen pour coupler le rayonnement laser de longueur d'ondes de base (λ₁) dans le moyen générant les autres longueurs d'ondes (λ₂, λ₃),
**caractérisé en ce que**
dans un état de fonctionnement à la fois le rayonnement laser de la longueur d'ondes de base (λ₁) et le rayonnement laser d'au moins une autre longueur d'ondes (λ₂, λ₃) peuvent être découplés du module de laser médical à plusieurs longueurs d'ondes,
les longueurs d'ondes (λ₂, λ₃) sont générées en fonction de la longueur d'ondes (λ₁),
la première source de rayonnement laser (1) est une diode laser, le moyen (2) pour générer la longueur d'ondes (λ₂) est un laser solide et le moyen (3) pour générer la longueur d'ondes (λ₃) est un doubleur de fréquence non linéaire et
par une disposition appropriée du rayonnement au moins une composante du groupe des longueurs d'ondes (λ₁, λ₂, λ₃) peut être extraite séparément ou en combinaison totale à l'aide d'un système de transmission de lumière du module de traitement à laser médical.

2. Module de traitement à laser médical selon la revendication 1,
**caractérisé en ce que**
pour générer le rayonnement laser de longueur d'ondes (λ₂) le laser solide génère une longueur d'ondes plus longue que la longueur d'ondes (λ₁) et pour générer un rayonnement laser de longueur d'ondes (λ₃) le doubleur de fréquence non linéaire génère une longueur d'ondes plus courte.

3. Module de traitement à laser médical selon l'une ou les deux revendications 1 et 2,
**caractérisé en ce que**
le milieu actif du laser à diodes est une composante du groupe de l'arséniure de gallium (GaAs), de l'arséniure indium-gallium (InGaAs), de l'arséniure gallium-aluminium (GaAlAs), de l'arséniure indium-gallium-aluminium (InGaAlAs) ou d'un phosphite indium-gallium-arséniure (InGaAsP).

4. Module de traitement à laser médical selon l'une ou plusieurs des revendications précédentes,
**caractérisé en ce que**
la diode laser permet de générer de la lumière d'une longueur d'ondes dans la plage des longueurs d'ondes (λ₁) comprises entre 900 nm et 1000 nm.

5. Module de traitement à laser médical selon une ou plusieurs des revendications précédentes,
**caractérisé en ce que**
plusieurs diodes laser sont réparties suivant un réseau de diodes laser.

6. Module de traitement à laser médical selon l'une ou plusieurs des revendications précédentes,
**caractérisé en ce que**
le laser solide comporte un cristal à activité laser choisi dans le groupe comprenant Nd:YAG, Nd:YLF, Ho:YAG, Er:YAG, ErCr:YSGG, Er:GGG, Er:YSGG, Er:YLF, CrTmEr:YAG ou un cristal dopé avec d'autres terres rares.

7. Module de traitement à laser médical selon l'une ou plusieurs des revendications précédentes,
**caractérisé en ce que**
le laser solide est apte à générer une longueur d'ondes dans une plage comprise entre 1,5 µm et 3 µm.

8. Module de traitement à laser médical selon l'une des revendications 6 ou 7,
**caractérisé en ce que**
le cristal du laser à corps solide est placé dans une cavité permettant un pompage diffus.

9. Module de traitement à laser médical selon la revendication 8,
**caractérisé en ce que**
la cavité du laser solide est reliée par une conduite de liquide à la source de rayonnement laser (1),
la lumière de longueur d'ondes (λ₁) peut être injectée par le liquide dans la cavité pour pomper le cristal.

10. Module de traitement à laser médical selon la revendication 9,
**caractérisé en ce que**
la conduite de liquide est en forme de circuit traversé par un agrégat de refroidissement.

11. Module de traitement à laser médical selon l'une des revendications 9 ou 10,
**caractérisé en ce que**
la conduite de liquide et la cavité sont remplies de solution aqueuse, d'huile de silicone et/ou autres liquides appropriés.

12. Module de traitement à laser médical selon l'une ou plusieurs des revendications précédentes,
**caractérisé en ce que**
le doubleur de fréquence, non linéaire, comporte un cristal doubleur choisi dans le groupe comprenant KTP, KDP, LiNbO₃, KnbO₃, LiTaO₃ et LBO.

13. Module de traitement à laser médical selon l'une ou plusieurs des revendications précédentes,
**caractérisé en ce que**
le doubleur de fréquence, non linéaire, génère des longueurs d'ondes pour la plage (λ₃) comprises entre 450 nm et 500 nm.

14. Module de traitement à laser médical selon l'une ou plusieurs des revendications précédentes,
**caractérisé en ce que**
le doubleur de fréquence est équipé d'un résonateur pour amplifier sa longueur d'ondes d'émission (λ₃).

15. Module de traitement à laser médical selon l'une ou plusieurs des revendications 1 à 14,
**caractérisé en ce que**
le système de transmission de lumière est un guide de lumière par fibre optique.

16. Module de traitement à laser médical selon l'une ou plusieurs des revendications 1 à 15,
**caractérisé en ce qu'**
il est constitué par un module intégrable dans un appareil de traitement médical.

17. Instrument de traitement médical,
**caractérisé en ce qu'**
il comporte un module de traitement laser, médical, selon une ou plusieurs des revendications précédentes 1 à 16.

18. Instrument de traitement médical selon la revendication 17,
**caractérisé en ce qu'**
il s'agit d'un instrument dentaire.
